# EUROPEAN PATENT APPLICATION

(11) **EP 4 353 176 A1**
(43) Date of publication of application: **17.04.2024**
(21) Application number: 22212847.2
(22) Date of filing: 12.12.2022
(51) Int. Cl.: A61B 34/10

(54) **PROVIDING BIOMECHANICAL PLAQUE DATA FOR AN INTERVENTIONAL DEVICE SIMULATION SYSTEM**

(30) Priority: 11.10.2022 US 202263414994 P
(71) Applicant: Koninklijke Philips N.V., 5656 AG Eindhoven (NL)
(72) Inventor: HAASE, Hans Christian, Eindhoven (NL); GRASS, Michael, Eindhoven (NL); SCHMITT, Holger, 5656AG Eindhoven (NL); NICKISCH, Hannes, Eindhoven (NL); VAN DER HORST, Arjen, Eindhoven (NL); VEMBAR, Manindranath, Eindhoven (NL)
(74) Representative: Philips Intellectual Property & Standards

(57) **Abstract**

A computer-implemented method of providing biomechanical plaque data (110) for an interventional device simulation system (100) includes: extracting plaque data from spectral CT data, the plaque data representing a spatial distribution of plaque (140) within a vascular region (130); converting the plaque data into biomechanical plaque data (110), the biomechanical plaque data representing a spatial distribution of a mechanical constraint to apply to an interventional device (150) of the interventional device simulation system (100) in response to a contact between the interventional device (150) and the plaque (140); and outputting the biomechanical plaque data (110).

## Description

### TECHNICAL FIELD

The present disclosure relates to providing biomechanical plaque data for an interventional device simulation system. A computer-implemented method, a computer program product, and a system, are disclosed.

### BACKGROUND

Interventional device simulation systems are becoming increasingly important in familiarizing physicians with the use of interventional devices. Simulation systems provide a virtual experience of interventional procedures that allows physicians to obtain experience for new intravascular procedures in settings that are both authentic, and also low-risk. For instance, in the vascular field, simulation systems have been developed and integrated into real cath. lab settings. Such systems are also used in the planning of patient-specific interventions.

An example of an interventional device simulation system in the vascular field is the Vist G5 endovascular simulator marketed by Mentice AB, Gothenburg, Sweden. Another example of an interventional device simulation system in the vascular field is the Angio Mentor system by Simbionix Ltd., Israel, and which is marketed by Surgical Science Sweden AB, Gothenburg, Sweden. Such systems provide both visual and tactile feedback, authentically mimicking the look and feel of actual endovascular interventions. Many different types of endovascular procedures may be simulated using such systems. Simulations may be performed with different types of interventional devices, and in different types of patient anatomies. For instance, navigation procedures such as catheter insertion may be simulated. Treatment procedures such as atherectomy procedures may also be simulated.

An important factor in providing authentic simulations in the vasculature is to accurately represent vascular plaque. Vascular plaque is formed from materials such as fat, cholesterol, calcium, fibrin, and other substances that build-up in the walls of arteries. Plaque causes the arteries to harden and narrow, restricting the blood flow and supply of oxygen to vital organs, increasing the risk of blood clots that could potentially block the flow of blood to the heart or brain. Consequently, the presence of vascular plaque affects interventional device navigation, vessel deformation, and also treatment decisions. However, a correlation between simulated procedures and real procedures, and also any benefit that is derived therefrom, is only feasible if the properties of the plaque are accurately represented in the simulations.

### SUMMARY

According to one aspect of the present disclosure, a computer-implemented method of providing biomechanical plaque data for an interventional device simulation system, is provided. The method includes:
receiving spectral CT data representing a vascular region, the spectral CT data defining X-ray attenuation within the vascular region in a plurality of different energy intervals;
extracting plaque data from the spectral CT data, the plaque data representing a spatial distribution of plaque within the vascular region;
converting the plaque data into biomechanical plaque data, the biomechanical plaque data representing a spatial distribution of a mechanical constraint to apply to an interventional device of the interventional device simulation system in response to a contact between the interventional device and the plaque; and
outputting the biomechanical plaque data.

Spectral CT data defines X-ray attenuation in a plurality of different energy intervals. By processing the data from the multiple different energy intervals, a distinction can be made between media that have similar X-ray attenuation values when measured within a single energy interval, and which would be indistinguishable in conventional X-ray attenuation data. Since, in the above method, the plaque data is extracted from spectral CT data, the plaque data provides a more accurate representation of the spatial distribution of the plaque. Moreover, since the plaque data is converted into biomechanical plaque data that represents a spatial distribution of a mechanical constraint to apply to an interventional device of the interventional device simulation system in response to a contact between the interventional device and the plaque, a more accurate simulation of the interaction between the interventional device and the plaque, is provided.

Further aspects, features, and advantages of the present disclosure will become apparent from the following description of examples, which is made with reference to the accompanying drawings.

### BRIEF DESCRIPTION OF THE DRAWINGS

Fig. 1 is an example of a graphical representation of a vascular region 130, in accordance with some aspects of the present disclosure.
Fig. 2 is a schematic diagram illustrating an example of a spatial distribution of plaque 140 within a vascular region, in accordance with some aspects of the present disclosure.
Fig. 3 is a flowchart illustrating an example of a computer-implemented method of providing biomechanical plaque data for an interventional device simulation system, in accordance with some aspects of the present disclosure.
Fig. 4 is a schematic diagram illustrating an example of a system 200 for providing biomechanical plaque data for an interventional device simulation system, in accordance with some aspects of the present disclosure.
Fig. 5 is a schematic diagram illustrating an example of an interventional device simulation system 100, in accordance with some aspects of the present disclosure.
Fig. 6 is a flowchart illustrating an example of a computer-implemented method of simulating a navigation of an interventional device within a vascular region, in accordance with some aspects of the present disclosure.
Fig. 7 is a flowchart illustrating an example of a computer-implemented method of providing guidance for a current interventional procedure involving a navigation of an interventional device within a vascular region, in accordance with some aspects of the present disclosure.
Fig. 8 is a flowchart illustrating an example of a computer-implemented method of identifying a type of interventional device to use in a current interventional procedure, in accordance with some aspects of the present disclosure.
Fig. 9 is a flowchart illustrating an example of a computer-implemented method of providing a predictive model for controlling a robotic interventional device manipulator to navigate an interventional device within a vascular region, in accordance with some aspects of the present disclosure.
Fig. 10 is a flowchart illustrating an example of a computer-implemented method of simulating a robotic navigation of an interventional device within a vascular region during a current interventional device navigation procedure using an interventional device simulation system, in accordance with some aspects of the present disclosure.
Fig. 11 is a flowchart illustrating an example of a computer-implemented method of determining a difference between a simulated robotic interventional device navigation procedure and a current interventional device navigation procedure, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to computer-implemented methods that involve providing biomechanical plaque data for an interventional device simulation system. Reference is made to examples in which the biomechanical plaque data is provided for a vascular region in the heart. However, it is to be appreciated that the vascular region may in general be in any location in the body. For instance, the vascular region may be in the brain, an arm, a leg, and so forth. In some examples, reference is made to the provision of biomechanical plaque data for an artery. However, it is noted that the biomechanical plaque data may in general be provided for any vessel in the vasculature. For instance, the biomechanical plaque data may alternatively be provided for a vein. In some examples, reference is made to the provision of biomechanical plaque data for a mature plaque. However, it is to be appreciated that this type of plaque serves only as an example, and that the methods disclosed herein may be used to provide biomechanical plaque data for a plaque that is in a different stage of maturity. For instance, the plaque may be a so-called fatty streak, or a ruptured plaque. Thus, the methods disclosed herein may also be used to provide biomechanical plaque data for plaque that has a different composition to mature plaque.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

As mentioned above, a correlation between simulated procedures and real procedures, and also any benefit that is derived therefrom, is only feasible if the properties of the plaque are accurately represented in the simulations.

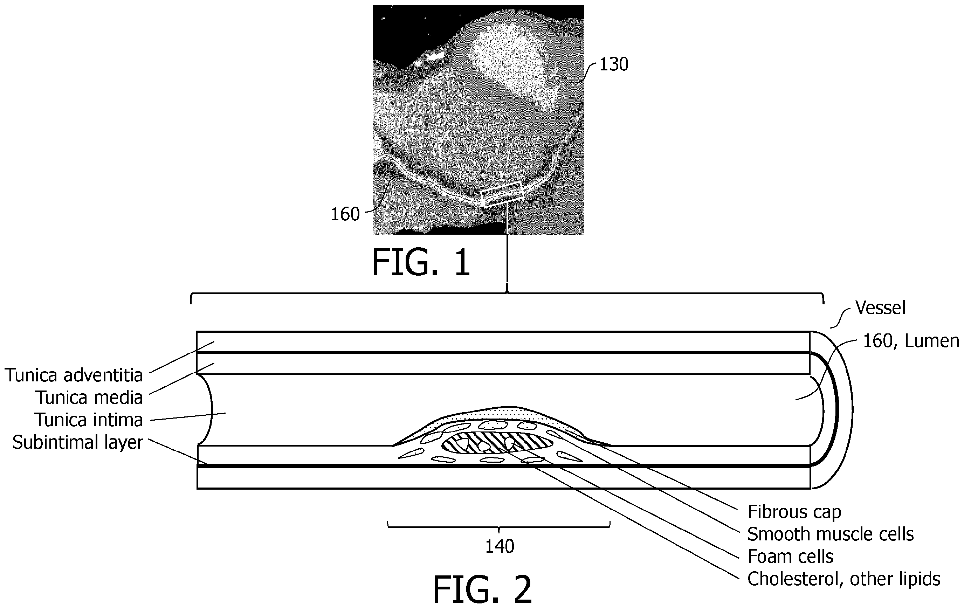

Fig. 1 is an example of a graphical representation of a vascular region 130, in accordance with some aspects of the present disclosure. The vascular region 130 represented in Fig. 1 is a portion of the heart, and the vascular region includes a coronary artery. The lumen 160 of the coronary artery is labelled in Fig. 1. Fig. 2 is a schematic diagram illustrating an example of a spatial distribution of plaque 140 within a vascular region, in accordance with some aspects of the present disclosure. The spatial distribution of the plaque 140 illustrated in Fig. 2 represents a plaque in a portion of the coronary artery illustrated in Fig. 1, as indicated by the line connecting Fig. 1 and Fig. 2. The plaque 140 illustrated in Fig. 2 is a so-called mature plaque, and includes a fibrous cap. The fibrous cap is formed of intimal smooth muscle cells and connective tissue. The fibrous cap separates the lumen 160 of the coronary artery from the thrombogenic core of the plaque, and as such is the final barrier to thrombus formation. The core of the plaque illustrated in Fig. 2 includes foam cells, cholesterol, and other lipids.

Over time, the composition, and also distribution, of a plaque in a vessel can change. For instance, the plaque may start with a so-called fatty streak, and subsequently evolve into the mature plaque illustrated in Fig. 1. The mature plaque may subsequently evolve further by rupturing, resulting in a thrombus. During its evolution, vascular calcifications can also occur in the vessel 130. Vascular calcification is defined as mineral deposition in the vasculature in the form of calcium-phosphate complexes. Vascular calcification can occur in the intimal and medial layers.

Thus, the spatial distribution of plaque, as well as its composition depends on the stage of its evolution. This in turn affects its biomechanical properties, i.e. the mechanical properties that govern its response to the application of an external force. Consequently, in order to accurately represent the interaction between an interventional device and the plaque, it is important that the spatial distribution of the biomechanical properties of the plaque, are accurately represented.

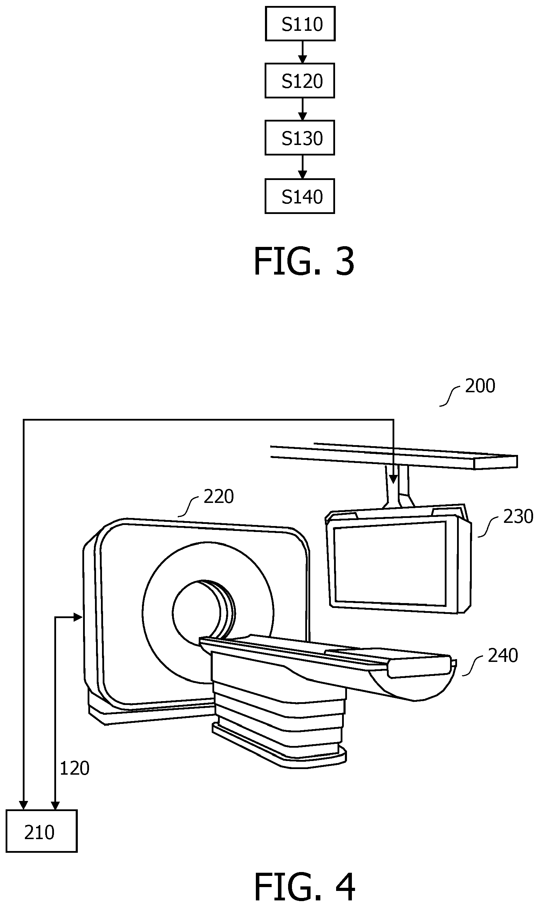

Fig. 3 is a flowchart illustrating an example of a computer-implemented method of providing biomechanical plaque data for an interventional device simulation system, in accordance with some aspects of the present disclosure. Fig. 4 is a schematic diagram illustrating an example of a system 200 for providing biomechanical plaque data for an interventional device simulation system, in accordance with some aspects of the present disclosure. The system 200 includes one or more processors 210. It is noted that operations described in relation to the method illustrated in Fig. 3, may also be performed by the one or more processors 210 of the system 200 illustrated in Fig. 4. Likewise, operations described in relation to the one or more processors 210 of the system 200, may also be performed in the method described with reference to Fig. 3. With reference to Fig. 3, the computer-implemented method of providing biomechanical plaque data 110 for an interventional device simulation system 100, includes:
receiving S110 spectral CT data 120 representing a vascular region 130, the spectral CT data defining X-ray attenuation within the vascular region in a plurality of different energy intervals DE_{1..m};
extracting S120 plaque data from the spectral CT data 120, the plaque data representing a spatial distribution of plaque 140 within the vascular region 130;
converting S130 the plaque data into biomechanical plaque data 110, the biomechanical plaque data representing a spatial distribution of a mechanical constraint to apply to an interventional device 150 of the interventional device simulation system 100 in response to a contact between the interventional device 150 and the plaque 140; and
outputting S140 the biomechanical plaque data 110.

Spectral CT data defines X-ray attenuation in a plurality of different energy intervals. By processing the data from the multiple different energy intervals, a distinction can be made between media that have similar X-ray attenuation values when measured within a single energy interval, and which would be indistinguishable in conventional X-ray attenuation data. Since, in the above method, the plaque data is extracted from spectral CT data, the plaque data provides a more accurate representation of the spatial distribution of the plaque. Moreover, since the plaque data is converted into biomechanical plaque data that represents a spatial distribution of a mechanical constraint to apply to an interventional device of the interventional device simulation system in response to a contact between the interventional device and the plaque, a more accurate simulation of the interaction between the interventional device and the plaque, is provided.

With reference to the method illustrated in Fig. 3, in the operation S110 spectral CT data 120 is received. The spectral CT data represents a vascular region 130, and defines X-ray attenuation within the vascular region in a plurality of different energy intervals DE_{1..m}.

In general, the spectral CT data 120 that is received in the operation S110 may be raw data, i.e. data that has not yet been reconstructed into a volumetric, or 3D, image, or it may be image data, i.e. data that has already been reconstructed into a volumetric image. In general there may be two or more energy intervals; i.e. *m* is an integer, and *m* ≥ 2.

The spectral CT data 120 that is received in the operation S110 may be generated by a spectral CT imaging system, or, as described in more detail below, it may alternatively be generated by rotating, or stepping the X-ray source and X-ray detector of a spectral X-ray projection imaging system around the vascular region. More generally, the spectral CT data 120 that is received in the operation S110 may be generated by a spectral X-ray imaging system.

A spectral CT imaging system generates spectral CT data by rotating, or stepping, an X-ray source-detector arrangement around an object and acquiring X-ray attenuation data for the object from multiple rotational angles with respect to the object. The spectral CT data may then be reconstructed into a 3D image of the object. Examples of spectral CT imaging systems that may be used to generate the spectral CT data 120 include cone beam spectral CT imaging systems, photon counting spectral CT imaging systems, dark-field spectral CT imaging systems, and phase contrast spectral CT imaging systems. An example of a spectral CT imaging system that may be used to generate spectral CT data 120 that is received in the operation S110 is the Spectral CT 7500 that is marketed by Philips Healthcare, Best, The Netherlands.

As mentioned above, the spectral CT data 120 that is received in the operation S 110 may alternatively be generated by rotating, or stepping the X-ray source and X-ray detector of a spectral X-ray projection imaging system around the vascular region. A spectral X-ray projection imaging system typically includes a support arm such as a so-called "C-arm" that supports an X-ray source and an X-ray detector. Spectral X-ray projection imaging systems may alternatively include a support arm with a different shape to this example, such as an O-arm, for example. In contrast to a spectral CT imaging system, a spectral X-ray projection imaging system generates X-ray attenuation data for an object with the X-ray source and X-ray detector in a static position with respect to the object. The X-ray attenuation data may be referred-to as projection data, in contrast to the volumetric data that is generated by a spectral CT imaging system. The X-ray attenuation data that is generated by a spectral X-ray projection imaging system is typically used to generate a 2D image of the object. However, a spectral X-ray projection imaging system may generate spectral CT data, i.e. volumetric data, by rotating, or stepping, its X-ray source and X-ray detector around an object and acquiring projection data for the object from multiple rotational angles with respect to the object. Image reconstruction techniques may then be used to reconstruct the projection data that is obtained from the multiple rotational angles into a volumetric image in a similar manner to the reconstruction of a volumetric image using X-ray attenuation data that is acquired from a spectral CT imaging system. Thus, the spectral CT data 120 that is received in the operation S 110, may be generated by a spectral CT imaging system, or alternatively, it may be generated by a spectral X-ray projection imaging system.

The ability to generate X-ray attenuation data in multiple different energy intervals DE_{1..m} distinguishes a spectral X-ray imaging system from a conventional X-ray imaging system. By processing the data from the multiple different energy intervals, a distinction can be made between media that have similar X-ray attenuation values when measured within a single energy interval, and which would be indistinguishable in conventional X-ray attenuation data.

In general, the spectral CT data 120 may be generated by various different configurations of spectral X-ray imaging systems that include an X-ray source and an X-ray detector. The X-ray source of a spectral X-ray imaging system may include multiple monochromatic sources, or one or more polychromatic sources, and the X-ray detector of a spectral X-ray imaging system may include: a common detector for detecting multiple different X-ray energy intervals, or multiple detectors wherein each detector detects a different X-ray energy interval DE_{1..m}, or a multi-layer detector in which X-rays having energies within different X-ray energy intervals are detected by corresponding layers, or a photon counting detector that bins detected X-ray photons into one of multiple energy intervals based on their individual energies. In a photon counting detector, the relevant energy interval may be determined for each received X-ray photon by detecting the pulse height induced by electron-hole pairs that are generated in response to the X-ray photon's absorption in a direct-conversion material.

Various configurations of the aforementioned X-ray sources and detectors may be used to detect X-rays within different X-ray energy intervals DE_{1..m}. In general, discrimination between different X-ray energy intervals may be provided at the source by temporally switching the X-ray tube potential of a single X-ray source, i.e. "rapid kVp switching", or by temporally switching, or filtering, the emission of X-rays from multiple X-ray sources. The temporal switching may be performed within a rotation of the X-ray source-detector arrangement such that X-ray attenuation data for multiple different X-ray energy intervals DE_{1..m}, is acquired within a rotation; or alternatively, X-ray attenuation data for an energy interval may be acquired for an X-ray energy interval during a specified number of rotations of the gantry before switching to another energy interval and similarly acquiring X-ray attenuation data for that energy interval. In such configurations, a common X-ray detector may be used to detect X-rays across multiple different energy intervals, X-ray attenuation data for each energy interval being generated in a time-sequential manner. Alternatively, discrimination between different X-ray energy intervals may be provided at the detector by using a multi-layer detector, or a photon counting detector. Such detectors can detect X-rays from multiple X-ray energy intervals DE_{1..m} near-simultaneously, and thus there is no need to perform temporal switching at the source. Thus, a multi-layer detector, or a photon counting detector, may be used in combination with a polychromatic source to generate X-ray attenuation data at different X-ray energy intervals DE_{1..m}.

Other combinations of the aforementioned X-ray sources and detectors may also be used to provide the spectral CT data 120. For example, in a yet further configuration, the need to sequentially switch different X-ray sources emitting X-rays at different energy intervals may be obviated by mounting X-ray source-detector pairs to a gantry at rotationally-offset positions around an axis of rotation. In this configuration, each source-detector pair operates independently, and separation between the spectral CT data for different energy intervals DE_{1..m} is facilitated by virtue of the rotational offsets of the source-detector pairs. Improved separation between the spectral CT data for different energy intervals DE_{1..m} may be achieved with this configuration by applying an energy-selective filter to the X-ray detector(s) in order to reduce the effects of X-ray scatter.

In general, the spectral CT data 120 that is received in the operation S 110 may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals. The spectral CT data 120 that is received in the operation S110 may be received from various sources. For example, the spectral CT data 120 may be received from a spectral X-ray imaging system, such as one of the spectral X-ray imaging systems described above. Alternatively, the spectral CT data 120 may be received from another source, such as a computer readable storage medium, the Internet, or the Cloud, for example.

Returning to the method illustrated in Fig. 3, in the operation S120, plaque data is extracted from the spectral CT data 120. The plaque data represents a spatial distribution of plaque 140 within the vascular region 130.

Various material decomposition techniques may be used in the operation S120 to extract the plaque data. These include the use of various material decomposition algorithms, and the generation of a so-called "Z_{eff} image" that represents the effective atomic number of media, and from which various materials may be identified.

One example of a material decomposition technique that may be used to extract the plaque data in the operation S120 is disclosed in a document by Brendel, B. et al., "Empirical, projection-based basis-component decomposition method", Medical Imaging 2009, Physics of Medical Imaging, edited by Ehsan Samei and Jiang Hsieh, Proc. of SPIE Vol. 7258, 72583Y. Another example of a material decomposition algorithm that may be used is disclosed in a document by Roessl, E. and Proksa, R., "Kedge imaging in X-ray computed tomography using multi-bin photon counting detectors", Phys Med Biol. 2007 Aug 7, 52(15):4679-96. Another example of a material decomposition algorithm that may be used is disclosed in the published PCT patent application WO/2007/034359 A2. These, and other material decomposition algorithms may be used to extract from the spectral CT data 120, plaque data for different types of plaque, such as soft plaque, calcified plaque, and so forth.

As mentioned above, in another approach, a so-called "Z_{eff} image" that represents the effective atomic number of media may be used to extract the plaque data in the operation S120. An example of a technique for generating a Z_{eff} image is disclosed in a document by Saito, M., et al., "A simple formulation for deriving effective atomic numbers via electron density calibration from dual-energy CT data in the human body", Medical Physics, Vol. 44, Issue 6, June 2017, pages 2293-2303. Having generated a Z_{eff} image, the data for a desired material may be extracted from the image by selecting the data within a range of effective atomic numbers that corresponds to the material. For example, plaque data for calcified plaque, or for soft plaque, may be extracted from the spectral CT data by selecting data from the Z_{eff} image that is within the relevant range of effective atomic numbers that corresponds to calcified plaque, or soft plaque, respectively.

Returning to the method illustrated in Fig. 3, in the operation S130, the plaque data is converted into biomechanical plaque data 110. The biomechanical plaque data represents a spatial distribution of a mechanical constraint to apply to an interventional device 150 of the interventional device simulation system 100 in response to a contact between the interventional device 150 and the plaque 140.

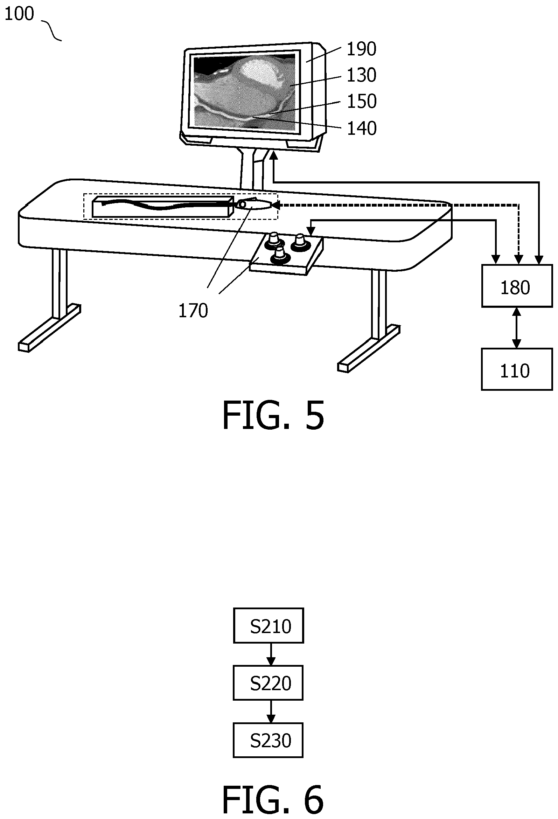

Fig. 5 is a schematic diagram illustrating an example of an interventional device simulation system 100, in accordance with some aspects of the present disclosure. The interventional device simulation system 100 illustrated in Fig. 5 includes a user interface device 170 and one or more processors 180. The user interface device 170 generates, in response to received user input, user input data for guiding an interventional device 150 within a vascular region 130. The one or more processors 180 determine a virtual position of the interventional device 150 within the vascular region based on the user input data generated by the user interface device 170. The vascular region is defined by a 3D mathematical model, and consequently the position of the interventional device within the vascular region is a "virtual" position. The virtual position of the interventional device 150 within the vascular region is then displayed on a display device, such as on the monitor 190 illustrated in Fig. 5. By facilitating a user to navigate an interventional device in the vasculature, the user may gain experience of various interventional procedures using the interventional device simulation system 100 illustrated in Fig. 5.

During the navigation of the interventional device in the vascular region, the interventional device often makes contact with plaque. The contact is "virtual" in the sense that since the position of the interventional device in the vascular region is virtual, the contact with the plaque is also virtual. As mentioned above, the spatial distribution of plaque, as well as its composition depends on the stage of its evolution. This in turn affects its biomechanical properties, i.e. the mechanical properties that govern its response to the application of an external force. Consequently, in order to accurately represent the interaction between an interventional device and the plaque, it is important that the spatial distribution of the biomechanical properties of the plaque, are accurately represented.

In the operation S130, the plaque data is converted into biomechanical plaque data 110. The biomechanical plaque data may represent a spatial distribution of one or more mechanical constraints to apply to an interventional device 150 of the interventional device simulation system 100 in response to a contact between the interventional device 150 and the plaque 140.

An example of a mechanical constraint that may be applied to an interventional device 150 in the operation S130 is a spatial limitation. In this example, the spatial limitation is applied a position of the interventional device 150 in response to the contact between the interventional device 150 and the plaque 140. The spatial limitation may define that the contacting portion of the interventional device cannot go beyond a surface of the plaque. Thus, the spatial limitation affects the freedom of movement of the interventional device. The spatial limitation may also vary depending on an amount of force applied to the plaque by the interventional device. This may be used to model a deformation of the surface of the plaque in response to the force, whereupon a new spatial limitation is defined based on the deformed surface of the plaque. The deformation of the surface of the plaque may also be modelled so as to represent the rupture of the plaque in response to excessive force, whereupon a further spatial limitation is defined based on an expected shape of the ruptured plaque. If the plaque data that is extracted from the spectral CT data 120 in the operation S110 distinguishes between different types of plaque, the amount of deformation may be calculated for the relevant type of plaque.

In general, the amount of deformation of the surface of the plaque may be modelled using the mechanical properties of the plaque. In this regard, the amount of deformation of the surface of the plaque may be modelled using mechanical properties of the plaque such as its elasticity, plasticity, ductility, malleability, hardness, toughness, brittleness, tenacity, fatigue, fatigue resistance, impact resistance property, strength, strain energy, resilience, proof resilience, modulus of resilience, creep, rupture, and modulus of toughness. One way of expressing the mechanical properties of the plaque is via its stress-strain curve. The stress-strain curve for a material governs the amount of deformation of the material when an external force is applied. Thus, a stress-strain curve for the plaque may be used to model the amount of deformation of the surface of the plaque in response to a force applied the plaque by the interventional device at the point of contact. The modelled deformation of the plaque surface then presents a new spatial limitation that is applied a position of the interventional device 150 in response to the contact between the interventional device 150 and the plaque 140. If the fracture point on the stress-strain curve is exceeded by applying excessive force, the plaque can be expected to fracture. This effect may also be modelled using the stress-strain curve by applying a further spatial limitation that is defined based on an expected shape of the ruptured plaque if the fracture point on the stress-strain curve is exceeded. In a simulation this event would be a significant performance indicator that is recorded and possibly leads to a visual notification.

Another example of a mechanical constraint that may be applied to an interventional device 150 in the operation S130 is friction. In this example, an amount of friction is applied to the interventional device 150 in response to a contact between the interventional device 150 and the plaque 140. Friction reduces the mobility of the interventional device where it contacts the plaque. In turn, friction may cause the interventional device to bend, which in turn affects its movement in the vascular region. The amount of friction may be calculated using the coefficient of friction for the plaque, for example.

Another example of a mechanical constraint that may be applied to an interventional device 150 in the operation S130 is an amount of force feedback to apply to a force feedback user interface device of the simulation system in response to the contact between the interventional device 150 and the plaque 140. Some user interface devices of simulation systems include so-called force feedback wherein the user experiences an opposing force in response to contact between the interventional device 150 and media in the vasculature. With such user interfaces, a user may experience an opposing force in response to attempts to e.g. force a guidewire against an obstruction in the vasculature. Force feedback improves the authenticity of a user's training experience. With such user interfaces, the amount of force feedback that is applied is determined by modelling the mechanical properties of the interventional device. In this example, the mechanical constraint that is applied, i.e. the amount of force feedback, is determined by further taking into account the spatial limitation described above. In other words, the spatial limitation provided by the plaque data, and in which a deformation of the plaque may be modelled, and a rupture of the plaque may further be modelled, is used to determine the amount of force feedback that is applied to the force feedback user interface device. Consequently, so-called soft plaques may feel softer to a user of the user interface device than hard plaques. Moreover, the rupture of a plaque may also be experienced by a user of force feedback user interface device as a change in the amount force feedback.

In general, the operation S130 of converting the plaque data into biomechanical plaque data 110 may be performed using a functional relationship between the plaque data and the mechanical constraint. The functional relationship may be provided by a graph, or a lookup table, for example.

By way of an example, the plaque data that is extracted from the spectral CT data 120 may represent a spatial distribution of plaque in two categories: hard plaque, and soft plaque. A functional relationship in the form of a graph representing the relationship between stress and strain may be provided for soft plaque, and anther graph may be provided for hard plaque. In response to contact between the interventional device and the plaque, the stress-strain graph for the relevant soft/ hard plaque is queried based on the amount of force exerted on the plaque by the interventional device, in order to model the amount of deformation induced in the plaque. This deformation is then used to modify the spatial limitation that is applied to the position of the interventional device by modifying the plaque data such that the shape of the plaque represented by the plaque data is deformed in accordance with the deformation. If, based on the stress-strain graph, the amount of force exerted on the plaque by the interventional device is sufficient to fracture the plaque, this effect may also be represented in the modified plaque data by deforming the plaque such that the modified plaque data represents an estimated shape of the ruptured plaque.

By way of another example, a lookup table may be used to store the values for the coefficient of friction for each of the plaque categories: soft plaque, and hard plaque. In response to contact between the interventional device and the plaque, the lookup table for the relevant soft/ hard plaque is queried to determine the coefficient of friction to use. The coefficient of friction is then used to calculate an amount of friction that is applied as a constraint to the interventional device in response to a contact between the interventional device 150 and the plaque 140.

Returning to the method illustrated in Fig. 3, in the operation S140, the biomechanical plaque data 110 is outputted. The biomechanical plaque data 110 may be outputted in various ways. In one example, the biomechanical plaque data 110 is outputted to a computer-readable storage medium. In another example, a graphical representation of the biomechanical plaque data 110 is outputted to a display device. For instance, the graphical representation of the biomechanical plaque data 110 may be outputted to a monitor, or to another type of display device such as virtual reality headset, or an augmented reality headset. The graphical representation of the biomechanical plaque data 110 may be outputted to the display device as a 3D image, or as a 2D image. The 2D image may be generated by projecting the 3D image. The graphical representation of the biomechanical plaque data 110 may include a color coding, or a shading, or another type of formatting that depicts the plaque, and if extracted from the spectral CT data, the biomechanical plaque data 110 for different types of the plaque such as soft/ hard plaque may also be depicted. In another example, the biomechanical plaque data 110 is outputted to the Internet, or to the Cloud. In another example, the biomechanical plaque data 110 is outputted to a printer.

In one example, a graphical representation of the vascular region 130 is outputted, and the graphical representation of the vascular region 130 comprises a graphical representation of the plaque data and/or a graphical representation of the outputted biomechanical plaque data 110. In this example, the graphical representation of the vascular region 130 represents the vessels(s) in the vascular region. The graphical representation of the vascular region 130 may be generated from lumen data that is extracted from the spectral CT data 120 that is received in the operation S110. For instance, a material decomposition algorithm, or a Z_{eff} image, may be used to extract lumen data from the spectral CT data, and an image of the vessels(s) in the vascular region may be generated from the lumen data. This image may then be overlaid with graphical representation of the plaque data and/or the graphical representation of the outputted biomechanical plaque data 110. In a related example, the graphical representation may include a virtual angiographic projection of the vascular region 130. Such images may be used to facilitate a clinical diagnosis of the vascular region by a physician.

In another example, the lumen data for the vascular region is outputted. The lumen data may be used in combination with the biomechanical plaque data in simulations that are performed with the interventional device simulation system 100 illustrated in Fig. 5. For instance, the lumen data may be used to generate a 3D mathematical model representing the vascular region. Simulations may be performed with the lumen data in combination with the biomechanical plaque data wherein both the lumen data and the biomechanical plaque data provide spatial limitations that are applied to a position of an interventional device when the interventional device is navigated through the lumen(s) in the vascular region. In this example, the method described with reference to Fig. 3 includes:
extracting lumen data from the spectral CT data 120, the lumen data representing a three-dimensional shape of one or more lumens 160 within the vascular region 130; and
outputting the lumen data.

The lumen data may be extracted from the spectral CT data in a similar manner to the plaque data, i.e. by using a material decomposition technique. The lumen data may be outputted, in a similar manner to the biomechanical plaque data 110. In one example, the lumen data and the plaque data are outputted as a graphical representation. For instance, a 3D image may be generated from the lumen data, and the 3D image overlaid with a graphical representation of the biomechanical plaque data 110. By providing both the lumen data and the biomechanical plaque data 110 from the spectral CT data, personalized simulation data for the vascular region may be provided in which the two datasets are inherently registered to one another.

In another example, the method described with reference to Fig. 3 includes: calculating, from the plaque data, a spatial distribution of a risk of plaque rupture, and outputting a graphical representation of the risk of plaque rupture.

Since the plaque data is extracted from spectral CT data, the plaque data provides a more accurate depiction of the plaque than if plaque data were extracted from conventional CT data. For instance, the use of spectral CT data facilitates a determination of the type or plaque, and its composition, as described above. A high risk of rupture is associated with factors such as the presence of a fibrous cap to the plaque, as illustrated in Fig. 1. Thus, in this example, the risk of plaque rupture may be calculated by detecting such features in the plaque data. The graphical representation of the risk of plaque rupture may be outputted in various ways. For instance, it may be outputted as an overlay image on the biomechanical plaque data wherein a color coding, or a shading, or another type of formatting is used in the overlay to depict the risk of plaque rupture. The risk of plaque rupture may be used in addition to the biomechanical plaque data in simulations that are performed with the interventional device simulation system 100 illustrated in Fig. 5. For instance, the risk of plaque rupture may be used to simulate regions of the plaque that become ruptured as a result of contact between the interventional device and the plaque.

In another example, the computer-implemented method of providing biomechanical plaque data 110 for an interventional device simulation system 100, is performed by a system. The system 200 is illustrated in Fig. 4. The system 200 for providing biomechanical plaque data 110 for an interventional device simulation system 100 comprises one or more processors 210 configured to:
receive S110 spectral CT data 120 representing a vascular region 130, the spectral CT data defining X-ray attenuation within the vascular region in a plurality of different energy intervals DE_{1..m};
extract S120 plaque data from the spectral CT data 120, the plaque data representing a spatial distribution of plaque 140 within the vascular region 130;
convert S130 the plaque data into biomechanical plaque data 110, the biomechanical plaque data representing a spatial distribution of a mechanical constraint to apply to an interventional device 150 of the interventional device simulation system 100 in response to a contact between the interventional device 150 and the plaque 140; and
output S140 the biomechanical plaque data 110.

The system 200 may also include a spectral CT imaging system 220 for providing the spectral CT data 120. The system 200 may also include a display such as the monitor 230 illustrated in Fig. 4. The display may be used to displaying a graphical representation of the spectral CT data, the plaque data, the biomechanical plaque data 110, and so forth. The system 200 may also include a patient bed 240.

In another example, an interventional device simulation system 100 for simulating a navigation of an interventional device 150 within a vascular region 130, is provided. The simulation system includes:
a user interface device 170; and
one or more processors 180;
wherein the user interface device 170 is configured to generate, in response to received user input, user input data for guiding the interventional device 150 within the vascular region 130; and
wherein the one or more processors 180 are configured to:
   receive the lumen data and the biomechanical plaque data 110;
   determine a virtual position of the interventional device 150 within the one or more lumens 160 represented by the lumen data based on the user input data generated by the user interface device 170; and
   wherein the determining a virtual position of the interventional device 150 comprises applying to the interventional device 150 the mechanical constraint represented by the biomechanical plaque data 110 in response to a contact between the interventional device 150 and the plaque 140.

An example of the system 100 is illustrated in Fig. 5. The user interface 170 may be provided by various types of devices. For example, the user interface 170 may be include one or more input device such as: a joystick, a mouse, a touchpad, a keyboard, a rotational knob, a switch, a slider control, a gesture control device, and so forth. Such user interfaces generate user input data in the form of electrical, RF or optical signals using various sensors that are coupled to the user input device. The sensors may be provided by various types of sensors, including one or more switches, potentiometer, strain gauges, optical sensor, (depth) cameras, and so forth. In one example described below, a real interventional device forms part of the user interface device. In this example, various sensors such as those mentioned above may be used to generate user input data in response to a user's manipulations of the real interface device. In another example, the user interface is provided by a real controller of an interventional device. An example of such a controller is a catheter "handle" that is used in real procedures. This example is illustrated within the dashed outline in Fig. 5. A catheter handle includes controls in the form of switches, knobs, dials, and so forth that are used to advance, retract, and rotate the catheter, sometimes via one or more motors or actuators. Sensors may be used to monitor the status of the controls, and to thereby provide the user input data. The use of a real controller such as a catheter handle provides the user with a more authentic experience of an interventional procedure. In another example, the user interface device 170 applies force feedback to a user of the user interface device such that the user may experience, inter alia, an opposing force in response to contact between the interventional device 150 and media in the vasculature.

With continued reference to Fig. 5, the one or more processors 180 receive the lumen data and the biomechanical plaque data 110 described above. The one or more processors may receive the lumen data from a computer readable storage medium, or from the Internet, or the Cloud, for example. In one example, the system 100 is provided in the form of a training console that is used to deliver a training experience to a user, as illustrated in Fig 5. In this example the one or more processors 180 may form part of the training console. In another example, the one or more processors 180 are the same one or more processors 210 that are used to provide biomechanical plaque data for the interventional device simulation system, as described with reference to Fig. 4. Thus, in this example, the one or more processors 180 may also perform the operations of receiving S110 spectral CT data 120, extracting S120 plaque data from the spectral CT data 120, converting S130 the plaque data into biomechanical plaque data 110, and outputting S140 the biomechanical plaque data 110, that were described with reference to Fig. 3.

With continued reference to Fig. 5, the one or more processors 180 determine a virtual position of the interventional device 150 within the one or more lumens 160 represented by the lumen data based on the user input data generated by the user interface device 170.

In this operation, the lumen data that is extracted from the spectral CT data 120 is used to generate a 3D mathematical model of the one or more lumens 160, and the virtual position of the interventional device 150 is determined in the 3D mathematical model. Since both the lumen data and the biomechanical plaque data are extracted from the spectral CT data 120, a personalized model of the vascular region is provided in which the two datasets are inherently registered to one another. By way of an example, the 3D mathematical model may be generated from the spectral CT data using similar techniques as those disclosed for generating a model from conventional CT data in the document US 2012/197619 A1.

The position of the interventional device 150 within the one or more lumens 160 that is determined in this operation is a "virtual" position since the position is determined in a 3D mathematical model. The interventional device 150 is also virtual, and is provided by a 3D mathematical model that models a shape of the interventional device as it is navigated in the 3D mathematical model of the one or more lumens. The 3D mathematical model of the interventional device may be based on the mechanical properties of a real interventional device. For instance, the stiffness of a real interventional device may be modelled so as to provide the user with a more authentic experience of the interventional procedure. The virtual position of the interventional device 150 is determined by using the user input data to replicate the user's manipulations of the interventional device in the 3D mathematical model of the interventional device, and constraining the position and shape of the interventional device with the 3D mathematical model of the one or more lumens. Thus, the user interface device 170 is used to navigate the virtual interventional device within the one or more lumens based on the user input data generated by the user interface device 170. It is noted that although the interventional device is virtual in the sense that it is provided by a 3D mathematical model, the user interface device may include a "real" interventional device, as mentioned above. For instance, a real catheter may be provided for manipulation by a user, wherein the various sensors described above are used to monitor the movements of the catheter, and thereby generate user input data, which is used to replicate the movements of the real catheter in the 3D mathematical model of the catheter.

In general, the interventional device 150 may be any type of interventional device that is suitable for navigation in the vasculature. For example, depending on the type of interventional procedure that is simulated, the interventional device may be a guidewire, a catheter, an intravascular ultrasound "IVUS" imaging device, an optical coherence tomography "OCT" imaging device, a blood flow sensing device, a blood pressure monitoring device, a balloon catheter, a stent, an atherectomy device, and so forth.

With continued reference to Fig. 5, when there is contact between the interventional device 150 and the plaque 140 the one or more processors 180 determine the virtual position of the interventional device 150 within the one or more lumens represented by the lumen data by applying to the interventional device 150 the mechanical constraint represented by the biomechanical plaque data 110. In this operation, the mechanical constraints described above are applied to the interventional device. In other words, a spatial limitation may be applied to a position of the interventional device 150 in response to the contact between the interventional device 150 and the plaque 140. Alternatively or additionally, an amount of friction may be applied to the interventional device 150 in response to a contact between the interventional device 150 and the plaque 140. Alternatively or additionally, an amount of force feedback may be applied to a force feedback user interface device of the simulation system in response to the contact between the interventional device 150 and the plaque 140.

With continued reference to Fig. 5, a graphical representation of the vascular region, and which also includes the virtual position of the interventional device 150 within the one or more lumens 160, may then be displayed on a display such as the monitor 190 illustrated in Fig. 5. The graphical representation may alternatively be displayed on other types of display device such as virtual reality headset, or an augmented reality headset, for example. By facilitating a user to navigate an interventional device in the vasculature, the user may gain experience of various interventional procedures using the interventional device simulation system 100 illustrated in Fig. 5.

In one example, the system described with reference to Fig. 5 applies force feedback to a user of the force feedback user interface device. As described above, the use of force feedback facilitates a more authentic training experience. In this example, the user interface device 170 comprises a force feedback interface device, and the force feedback interface device is configured to receive control signals for applying force feedback to a user of the force feedback user interface device, and the mechanical constraint comprises an amount of force feedback to apply to the force feedback user interface device in response to the contact between the interventional device 150 and the plaque 140. In this example, the one or more processors 180 are configured to:
generate the control signals for applying the force feedback to the user of the user input device 170 based on the virtual position of the interventional device 150 within the one or more lumens 160; and
wherein the control signals generated by the one or more processors 180 in response to a contact between the interventional device 150 and a plaque 140 in the one or more lumens 160 are generated based on the biomechanical plaque data 110.

Thus, in this example, a virtual contact between the interventional device and the plaque results in force feedback being applied to the force feedback interface device. This may be used to provide an authentic experience in the case that the interventional device contacts, the plaque, or further deforms, or even ruptures the plaque.

In another example, the system described with reference to Fig. 5 outputs a graphical representation of the lumen data and the biomechanical plaque data 110. In this example, the one or more processors 180 are configured to output a graphical representation of the lumen data and the biomechanical plaque data 110. The graphical representation may be provided as an overlay image, for example.

In another example, the system described with reference to Fig. 5 is used to modify the biomechanical plaque data 110. In this example, the one or more processors 180 are further configured to receive input defining a modification to the biomechanical plaque data 110, and the control signals generated in response to a contact between the interventional device 150 and a plaque 140 in the one or more lumens 160, are determined based on the modified biomechanical plaque data 110.

In this example, the modification to the biomechanical plaque data may be provided via the user interface device 170. The modification to the biomechanical plaque data may define a modification to its mechanical properties, or its shape, for example. The input may be used to modify reference biomechanical plaque data so as to provide a user with a more complex, or a more straightforward simulation experience, for example. The input may be received via a menu, for example, wherein a user selects a level of complexity of the simulated procedure.

In another example, the system described with reference to Fig. 5 is used to modify the lumen data. In this example, the one or more processors 180 are configured to:
receive input defining a modification to the lumen data;
modify the lumen data based on the received input; and
wherein the determining a virtual position of the interventional device 150 within the one or more lumens 160 represented by the lumen data, is performed using the modified lumen data.

In this example, the modification to the lumen data may be provided via the user interface device 170. The modification to the lumen data may define a modification to a shape of the lumen. For instance, the lumen area, or the lumen curvature, may be changed in order to increase a level of complexity of the simulated procedure.

In a related example, the modification to the lumen data represents an inserted virtual implanted device in the one or more lumens 160. In this example the operation of determining a virtual position of the interventional device 150 comprises applying to the interventional device 150 the mechanical constraint based on a mechanical property of the virtual implanted device in response to a contact between the interventional device 150 and the virtual implanted device. In this example, the implanted device may be a stent, for example. The mechanical property of the stent may be its stiffness, or its coefficient of friction, for example. The stiffness of the stent, affects the lumen's deformation in response to contact from the interventional device. The coefficient of friction affects how the interventional device moves during contact with the stent. Thus, this example may be used to provide a more authentic simulation of the navigation of the interventional device past the stent.

In another example, a computer-implemented method of simulating a navigation of an interventional device 150 within a vascular region 130 using the interventional device simulation system 100 described with reference to Fig. 5, is provided. The method includes:
receiving S210 the lumen data and the biomechanical plaque data 110;
receiving S220 user input data generated by the user interface device 170;
determining S230 a virtual position of the interventional device 150 within one or more lumens 160 represented by the lumen data based on the user input data generated by the user interface device 170; and
wherein the determining a virtual position of the interventional device 150 comprises applying to the interventional device 150 the mechanical constraint represented by the biomechanical plaque data 110 in response to a contact between the interventional device 150 and the plaque 140.

The method is illustrated in Fig. 6, which is a flowchart illustrating an example of a computer-implemented method of simulating a navigation of an interventional device 150 within a vascular region 130, in accordance with some aspects of the present disclosure. It is noted that the operations described in relation to the system in Fig. 5 above, may also be performed in the method illustrated in Fig. 6.

In another example, the system described with reference to Fig. 5 is used to generate a database of procedure simulation data. In this regard, a computer-implemented method of generating a database of procedure simulation data using the interventional device simulation system 100, is provided. The method includes:
recording procedure simulation data, the procedure simulation data comprising for each simulated procedure:
the user input data generated by the user interface device 170;
   position data representing the virtual position of the interventional device 150 at each of a plurality of points in time;
the biomechanical plaque data 110 used in the simulated procedure;
device data representing a type of interventional device 150 used in the simulated procedure; and
outcome data for the simulated procedure; and
storing the procedure simulation data to a database.

In this example, the procedure simulation data may be recorded using, and also stored to, a computer readable storage medium. The user input data represents the input that is provided by the user, and may be recorded by storing the signals generated by the user interface device, for example. The signals may represent the movements of the joystick illustrated in Fig. 5, for example. The position data may be recorded by storing the virtual positions of the intervention device in the 3D mathematical model of the one or more lumens. The device data may indicate that the interventional device 150 is a catheter, or a guidewire, or another type of interventional device, for example. The outcome data may represent various factors such as one or more of: a duration of the simulated procedure, a success metric for the procedure, and a location in the one or more lumens 160 of one or more confounding factors encountered during the procedure.

The database of procedure simulation data that is generated using the system described with reference to Fig. 5 may be used for various purposes. In one example, the database of procedure simulation data is used to provide guidance for a current interventional procedure. In another example, the database of procedure simulation data is used to identify a type of interventional device 150 to use in a current interventional procedure. In another example, the database of procedure simulation data is used to train a predictive model to control a robotic interventional device manipulator.

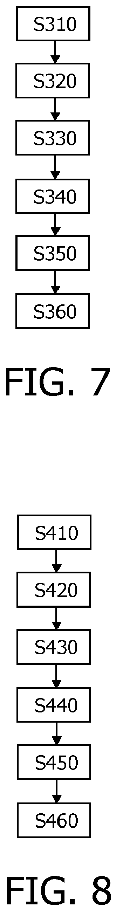

Fig. 7 is a flowchart illustrating an example of a computer-implemented method of providing guidance for a current interventional procedure involving a navigation of an interventional device within a vascular region, in accordance with some aspects of the present disclosure. With reference to Fig. 7, the method of providing guidance for a current interventional procedure involving a navigation of an interventional device 150 within a vascular region 130, includes:
receiving S310 the database of procedure simulation data;
receiving S320 spectral CT data 120 representing a vascular region 130 for the current interventional procedure;
extracting S330 plaque data for the current interventional procedure from the spectral CT data 120, the plaque data representing a spatial distribution of plaque 140 within the vascular region 130 for the current interventional procedure;
identifying S340 one or more similar simulated procedures from the database based on a similarity between the plaque data for the current interventional procedure and the plaque data for the simulated procedures;
predicting S350 an outcome metric for the current interventional procedure based on the outcome data for the identified one or more similar simulated procedures; and
outputting S360 the predicted outcome metric to provide the guidance for the current interventional procedure.

In this example, the operations of identifying S340, and predicting S350, and outputting S360, may be repeated during the current interventional procedure to provide an updated predicted outcome metric corresponding to a current time in the current interventional procedure.

In this example, the operations of receiving S320 spectral CT data 120, and extracting S330 plaque data, for the current interventional procedure, are performed in the same manner as described above with reference to Fig. 3. The similarity between the plaque data for the current interventional procedure, and the plaque data for the simulated procedures may be calculated based on factors such as a type of plaque identified in the procedures, and a distribution of the plaque identified in the procedures. The similarity between images may be calculated using metrics such as the Dice coefficient, the local root mean square error, and mutual information. The similarity between types, or classes of plaque, may be calculated by applying a weighted distance metric to the (multidimensional) class space. The outcome metric is predicted from the outcome data, which as mentioned above may represent one or more of: a duration of the simulated procedure, a success metric for the procedure, and a location in the one or more lumens 160 of one or more confounding factors encountered during the procedure. The prediction of the outcome metric may include selecting the outcome metric for the most similar simulated procedures from the database, or calculating the outcome metric as the average of multiple similar simulated procedures from the database, for example. By providing guidance for the current interventional procedure in accordance with this example, a physician may be assisted to carry out a more successful procedure.

As mentioned above, in another example, the database of procedure simulation data is used to identify a type of interventional device 150 to use in a current interventional procedure. This example is described with reference to Fig. 8, which is a flowchart illustrating an example of a computer-implemented method of identifying a type of interventional device to use in a current interventional procedure, in accordance with some aspects of the present disclosure. The computer-implemented method of identifying a type of interventional device 150 to use in a current interventional procedure, includes:
receiving S410 the database of procedure simulation data;
receiving S420 spectral CT data 120 representing a vascular region 130 for the current interventional procedure;
extracting S430 plaque data for the current interventional procedure from the spectral CT data 120, the plaque data representing a spatial distribution of plaque 140 within the vascular region 130 for the current interventional procedure;
identifying S440 one or more similar simulated procedures from the database based on a similarity between the plaque data for the current interventional procedure and the plaque data for the simulated procedures;
identifying S450 a type of interventional device 150 to use in the current interventional procedure based on the device data for the identified one or more similar simulated procedures; and
outputting S460 an indication of the identified type of the interventional device 150.

In this example, the operations of receiving S420 spectral CT data 120, and extracting S430 plaque data, for the current interventional procedure, are performed in the same manner as described above with reference to Fig. 3. The similarity between the plaque data for the current interventional procedure, and the plaque data for the simulated procedures may be calculated as described above with reference to Fig. 6. The device data represents the type of interventional device 150. The device data may indicate that the interventional device 150 is a catheter, or a guidewire, or another type of interventional device, for example. In this example, the operation of outputting S460 an indication of the identified type of the interventional device 150, may include outputting the device data for the most similar simulated procedures from the database, or determining the most common device data for multiple similar simulated procedures from the database and outputting the most common device data, for example.

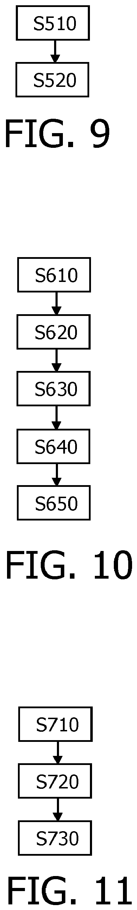

As mentioned above, in another example, the database of procedure simulation data is used to train a predictive model to control a robotic interventional device manipulator. This example is described with reference to Fig. 9, which is a flowchart illustrating an example of a computer-implemented method of providing a predictive model for controlling a robotic interventional device manipulator to navigate an interventional device within a vascular region, in accordance with some aspects of the present disclosure. With reference to Fig. 9, the computer-implemented method of providing a predictive model for controlling a robotic interventional device manipulator to navigate an interventional device 150 within a vascular region 130, includes:
receiving S510 the database of procedure simulation data; and
training S520 the predictive model to control the robotic interventional device manipulator to navigate the interventional device 150 within the vascular region 130 so as to replicate the position data representing the virtual position of the interventional device 150.

In this example, the robotic interventional device manipulator may be a handle of a catheter as described above. Alternatively the robotic interventional device manipulator may be another type of manipulator such as the manipulator of the Corindus CorPath GRX system that is marketed by Siemens Healthineers, Erlangen, Germany, or the R-One robotic assistance platform that is marketed by Robocath, Rouen, France. The latter two devices employ manipulators that can be used to navigate an elongate interventional device in the vasculature.

In this example, the database of procedure simulation data may include procedures for a variety of different spatial distributions of plaque, and a variety of compositions of the plaque. The predictive model in this example may be provided by a machine learning model, or by a neural network. Examples of suitable machine learning models include Random Forests, and shallow predictors such as support vector machines. Examples of suitable neural networks include convolutional neural networks "CNN", recurrent neural networks "RNN", or transformers.

In this example, the operation of training the predictive model may include controlling the user interface device 170 of the interventional device simulation system 100 via the robotic device manipulator, and wherein the controlling is based on an output of the predictive model. Thus, in this example, the robotic interventional device manipulator controls the user interface device 170 of the system 100 in order to provide a virtual position of the interventional device 150. The difference between the virtual position of the interventional device and the position of the interventional device in the procedure simulation data is used to train the predictive model.

In one example, the predictive model may be trained to control the robotic interventional device manipulator to navigate the interventional device 150 within the vascular region 130 using the procedure simulation data by:
receiving the procedure simulation data;
inputting the procedure simulation data into the predictive model; and
for each of a plurality of simulated procedures, and for each of a plurality of points in time:
   inputting the position data representing the virtual position of the interventional device 150 at a point in time from the simulated procedure;
   predicting a position of the interventional device 150 at a point in time, using the predictive model;
   adjusting parameters of the predictive model based on a difference between the predicted position of the interventional device 150 at the point in time and the virtual position of the interventional device 150 at a point in time; and
   repeating the predicting, and the adjusting, until a stopping criterion is met.

In another example, the neural network may be trained in an unsupervised manner. Reinforcement learning on simulated procedures allows an AI to train based on one or multiple success measures, independent of labelled ground truth data. The biomechanical plaque data that is used during the simulated training defines the specific clinical scenario the AI is trained for. The CT based plaque data from a clinical case is used to inform if such a trained AI is suited for a specific clinical case.

In general, the training of a neural network involves inputting a training dataset into the neural network, and iteratively adjusting the neural network's parameters until the trained neural network provides an accurate output. Training is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

The process of training the neural network described above therefore includes adjusting its parameters. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean absolute error (or L1 norm), the mean squared error, the root mean squared error (or L2 norm), the Huber loss, or the (binary) cross entropy loss. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers" These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

In another example, the procedure simulation data is grouped based on the type of plaque, and a separate predictive model is provided for different types of plaque. In this example, the method described with reference to Fig. 9 includes:
grouping the procedure simulation data based on a type of plaque represented by the biomechanical plaque data 110; and
wherein the method comprises providing a separate predictive model for controlling the robotic interventional device manipulator for each of a plurality of different types of plaque, each predictive model being trained using the procedure simulation data for the corresponding type of plaque.

Thus, different predictive models may be provided for different types of plaque.

In another example, a computer-implemented method of controlling a robotic interventional device manipulator to navigate an interventional device 150 within a vascular region 130 during a current interventional device navigation procedure, is provided. The method includes:
receiving a plurality of predictive models;
receiving spectral CT data 120 representing a vascular region 130 for the current interventional device navigation procedure;
extracting plaque data for the current interventional device navigation procedure from the spectral CT data 120, the plaque data representing a spatial distribution of plaque 140 within the vascular region 130 for the current interventional device navigation procedure;
selecting, from the predictive models, a predictive model based on a similarity between the plaque data for the current interventional device navigation procedure and the plaque data used to train the predictive model; and
controlling the robotic interventional device manipulator to navigate the interventional device 150 within the vascular region 130 during the current interventional device navigation procedure using the selected predictive model.

In this example, the plurality of predictive models that are received include separate predictive model for controlling the robotic interventional device manipulator for each of a plurality of different types of plaque. The operation of selecting a predictive model is based on a similarity between the plaque data for the current interventional device navigation procedure and the plaque data used to train the predictive model. The similarity between the plaque data for the current interventional procedure, and the plaque data used to train the predictive model may be calculated as described above with reference to Fig. 6. This example provides a predictive model for controlling a robotic interventional device manipulator to navigate an interventional device 150 in which the predictive model is suited to the plaque in the current interventional procedure.

In another example, the computer-implemented method of controlling a robotic interventional device manipulator to navigate an interventional device 150 within a vascular region 130 during a current interventional device navigation procedure, is performed by a system. Thus, a system for controlling a robotic interventional device manipulator to navigate an interventional device 150 within a vascular region 130 during a current interventional device navigation procedure, is provided. The system comprises one or more processors configured to:
receive a plurality of predictive models;
receive spectral CT data 120 representing a vascular region 130 for the current interventional device navigation procedure;
extract plaque data for the current interventional device navigation procedure from the spectral CT data 120, the plaque data representing a spatial distribution of plaque 140 within the vascular region 130 for the current interventional device navigation procedure;
select, from the predictive models, a predictive model based on a similarity between the plaque data for the current interventional device navigation procedure and the plaque data used to train the predictive model; and
control the robotic interventional device manipulator to navigate the interventional device 150 within the vascular region 130 during the current interventional device navigation procedure using the selected predictive model.

In another example, a computer-implemented method of simulating a robotic navigation of an interventional device 150 within a vascular region 130 during a current interventional device navigation procedure using the interventional device simulation system 100, is provided. This example is described with reference to Fig. 10, which is a flowchart illustrating an example of a computer-implemented method of simulating a robotic navigation of an interventional device within a vascular region during a current interventional device navigation procedure using an interventional device simulation system, in accordance with some aspects of the present disclosure. In this example, the method includes:
receiving S610 a plurality of predictive models;
receiving S620 spectral CT data 120 representing a vascular region 130 for the current interventional device navigation procedure;
extracting S630 plaque data for the current interventional device navigation procedure from the spectral CT data 120, the plaque data representing a spatial distribution of plaque 140 within the vascular region 130 for the current interventional device navigation procedure;
selecting 640, from the predictive models, a predictive model based on a similarity between the plaque data for the current interventional device navigation procedure and the plaque data used to train the predictive model; and
robotically S650 controlling the user interface device 170 of the interventional device simulation system 100 to navigate the interventional device 150 within the vascular region 130 during the current interventional device navigation procedure using the selected predictive model.

In this example, the plurality of predictive models that are received in the operation S610 include separate predictive model for controlling the robotic interventional device manipulator for each of a plurality of different types of plaque. By robotically S650 controlling the user interface device 170 of the interventional device simulation system 100 using the selected predictive model, a more optimal simulation is provided. This is because the selected model is an appropriate model for the type of plaque in the current interventional device navigation procedure. The simulation may be used to demonstrate to a physician a method of performing the interventional procedure.

In another example, a difference between a simulated robotic interventional device navigation procedure and a current interventional device navigation procedure, is determined. This may be used during a navigation procedure to determine when the current interventional device navigation deviates from an expected procedure. This example is described with reference to Fig. 11, which is a flowchart illustrating an example of a computer-implemented method of determining a difference between a simulated robotic interventional device navigation procedure and a current interventional device navigation procedure, in accordance with some aspects of the present disclosure. In this example, the method includes:
simulating S710 a robotic navigation of an interventional device 150 within a vascular region 130 during a current interventional device navigation procedure;
controlling S720 a robotic interventional device manipulator to navigate an interventional device 150 within a vascular region 130 during a current interventional device navigation procedure; and
determining S730 a difference between the simulated robotic navigation of the interventional device 150, and the navigation of the interventional device 150 performed by controlling the robotic interventional device manipulator to navigate the interventional device 150 within the vascular region 130 during the current interventional device navigation procedure.

In this example, the operation of simulating S710 a robotic navigation, is performed in accordance with the method illustrated in Fig. 10. The difference may be outputted in various ways. For instance, the difference may be outputted to a display device, or audially. In one example, a user is alerted via a message if the magnitude of the difference exceeds a threshold.

In another example, the computer-implemented method of determining a difference between a simulated robotic interventional device navigation procedure and a current interventional device navigation procedure, is performed by a system. Thus, a system for determining a difference between a simulated robotic interventional device navigation procedure and a current interventional device navigation procedure, is provided. The system comprises one or more processors configured to:
simulate S710 a robotic navigation of an interventional device 150 within a vascular region 130 during a current interventional device navigation procedure;
control S720 a robotic interventional device manipulator to navigate an interventional device 150 within a vascular region 130 during a current interventional device navigation procedure; and
determine S730 a difference between the simulated robotic navigation of the interventional device 150, and the navigation of the interventional device 150 performed by controlling the robotic interventional device manipulator to navigate the interventional device 150 within the vascular region 130 during the current interventional device navigation procedure.

The above examples are to be understood as illustrative of the present disclosure, and not restrictive. Further examples are also contemplated. For instance, the examples described in relation to computer-implemented methods, may also be provided by a computer program product, or by a computer-readable storage medium, or by a system, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of providing biomechanical plaque data (110) for an interventional device simulation system (100), the method comprising:
receiving (S110) spectral CT data (120) representing a vascular region (130), the spectral CT data defining X-ray attenuation within the vascular region in a plurality of different energy intervals (DE_{1..m});
extracting (S120) plaque data from the spectral CT data (120), the plaque data representing a spatial distribution of plaque (140) within the vascular region (130);
converting (S130) the plaque data into biomechanical plaque data (110), the biomechanical plaque data representing a spatial distribution of a mechanical constraint to apply to an interventional device (150) of the interventional device simulation system (100) in response to a contact between the interventional device (150) and the plaque (140); and
outputting (S140) the biomechanical plaque data (110).

2. The computer-implemented method according to claim 1, wherein the mechanical constraint comprises one or more of: a spatial limitation to apply to a position of the interventional device (150) in response to the contact between the interventional device (150) and the plaque (140) and/or an amount of friction to apply to the interventional device (150) in response to a contact between the interventional device (150) and the plaque (140) and/or an amount of force feedback to apply to a force feedback user interface device of the simulation system in response to the contact between the interventional device (150) and the plaque (140).

3. The computer-implemented method according to claim 1 or claim 2, further comprising:
outputting a graphical representation of the vascular region (130); and
wherein the graphical representation of the vascular region (130) comprises a graphical representation of the plaque data and/or a graphical representation of the outputted biomechanical plaque data (110).

4. The computer-implemented method according to any one of claims 1-3, wherein the method further comprises:
extracting lumen data from the spectral CT data (120), the lumen data representing a three-dimensional shape of one or more lumens (160) within the vascular region (130); and
outputting the lumen data.

5. The computer-implemented method according to any one of claims 1-4, wherein the method further comprises:
calculating, from the plaque data, a spatial distribution of a risk of plaque rupture; and
outputting a graphical representation of the risk of plaque rupture.

6. An interventional device simulation system (100) for simulating a navigation of an interventional device (150) within a vascular region (130), the simulation system comprising:
a user interface device (170); and
one or more processors (180);
wherein the user interface device (170) is configured to generate, in response to received user input, user input data for guiding the interventional device (150) within the vascular region (130); and
wherein the one or more processors (180) are configured to:
receive the lumen data and the biomechanical plaque data (110) outputted according to the method of claim 4;
determine a virtual position of the interventional device (150) within the one or more lumens (160) represented by the lumen data based on the user input data generated by the user interface device (170); and
wherein the determining a virtual position of the interventional device (150) comprises applying to the interventional device (150) the mechanical constraint represented by the biomechanical plaque data (110) in response to a contact between the interventional device (150) and the plaque (140).

7. The interventional device simulation system (100) according to claim 6, wherein the user interface device (170) comprises a force feedback interface device, and wherein the force feedback interface device is further configured to receive control signals for applying force feedback to a user of the force feedback user interface device, and wherein the mechanical constraint comprises an amount of force feedback to apply to the force feedback user interface device in response to the contact between the interventional device (150) and the plaque (140); and
wherein the one or more processors (180) are further configured to:
generate the control signals for applying the force feedback to the user of the user input device (170) based on the virtual position of the interventional device (150) within the one or more lumens (160); and
wherein the control signals generated by the one or more processors (180) in response to a contact between the interventional device (150) and a plaque (140) in the one or more lumens (160) are generated based on the biomechanical plaque data (110).

8. A computer-implemented method of simulating a navigation of an interventional device (150) within a vascular region (130) using the interventional device simulation system (100) according to claim 6, the method comprising:
receiving (S210) the lumen data and the biomechanical plaque data (110) outputted according to the method of claim 4;
receiving (S220) user input data generated by the user interface device (170);
determining (S230) a virtual position of the interventional device (150) within one or more lumens (160) represented by the lumen data based on the user input data generated by the user interface device (170); and
wherein the determining a virtual position of the interventional device (150) comprises applying to the interventional device (150) the mechanical constraint represented by the biomechanical plaque data (110) in response to a contact between the interventional device (150) and the plaque (140).

9. A computer-implemented method of generating a database of procedure simulation data using the interventional device simulation system (100) according to claim 6, the method comprising:
recording procedure simulation data, the procedure simulation data comprising for each simulated procedure:
the user input data generated by the user interface device (170);
position data representing the virtual position of the interventional device (150) at each of a plurality of points in time;
the biomechanical plaque data (110) used in the simulated procedure;
device data representing a type of interventional device (150) used in the simulated procedure; and
outcome data for the simulated procedure; and
storing the procedure simulation data to a database.

10. A computer-implemented method of providing guidance for a current interventional procedure involving a navigation of an interventional device (150) within a vascular region (130), the method comprising:
receiving (S310) the database of procedure simulation data generated according to the method of claim 9;
receiving (S320) spectral CT data (120) representing a vascular region (130) for the current interventional procedure;
extracting (S330) plaque data for the current interventional procedure from the spectral CT data (120), the plaque data representing a spatial distribution of plaque (140) within the vascular region (130) for the current interventional procedure;
identifying (S340) one or more similar simulated procedures from the database based on a similarity between the plaque data for the current interventional procedure and the plaque data for the simulated procedures;
predicting (S350) an outcome metric for the current interventional procedure based on the outcome data for the identified one or more similar simulated procedures; and
outputting (S360) the predicted outcome metric to provide the guidance for the current interventional procedure.

11. A computer-implemented method of providing a predictive model for controlling a robotic interventional device manipulator to navigate an interventional device (150) within a vascular region (130), the method comprising:
receiving (S510) the database of procedure simulation data generated according to the method of claim 9; and
training (S520) the predictive model to control the robotic interventional device manipulator to navigate the interventional device (150) within the vascular region (130) so as to replicate the position data representing the virtual position of the interventional device (150).

12. The computer-implemented method according to claim 11, wherein the method further comprises:
grouping the procedure simulation data based on a type of plaque represented by the biomechanical plaque data (110); and
wherein the method comprises providing a separate predictive model for controlling the robotic interventional device manipulator for each of a plurality of different types of plaque, each predictive model being trained using the procedure simulation data for the corresponding type of plaque.

13. A computer-implemented method of controlling a robotic interventional device manipulator to navigate an interventional device (150) within a vascular region (130) during a current interventional device navigation procedure, the method comprising:
receiving a plurality of predictive models provided according to the method of claim 12;
receiving spectral CT data (120) representing a vascular region (130) for the current interventional device navigation procedure;
extracting plaque data for the current interventional device navigation procedure from the spectral CT data (120), the plaque data representing a spatial distribution of plaque (140) within the vascular region (130) for the current interventional device navigation procedure;
selecting, from the predictive models, a predictive model based on a similarity between the plaque data for the current interventional device navigation procedure and the plaque data used to train the predictive model; and
controlling the robotic interventional device manipulator to navigate the interventional device (150) within the vascular region (130) during the current interventional device navigation procedure using the selected predictive model.

14. A computer-implemented method of simulating a robotic navigation of an interventional device (150) within a vascular region (130) during a current interventional device navigation procedure using the interventional device simulation system (100) according to claim 4, the method comprising:
receiving (S610) a plurality of predictive models provided according to the method of claim 12;
receiving (S620) spectral CT data (120) representing a vascular region (130) for the current interventional device navigation procedure;
extracting (S630) plaque data for the current interventional device navigation procedure from the spectral CT data (120), the plaque data representing a spatial distribution of plaque (140) within the vascular region (130) for the current interventional device navigation procedure;
selecting (640), from the predictive models, a predictive model based on a similarity between the plaque data for the current interventional device navigation procedure and the plaque data used to train the predictive model; and
robotically (S650) controlling the user interface device (170) of the interventional device simulation system (100) to navigate the interventional device (150) within the vascular region (130) during the current interventional device navigation procedure using the selected predictive model.

15. A computer-implemented method of determining a difference between a simulated robotic interventional device navigation procedure and a current interventional device navigation procedure, the method comprising:
simulating (S710) a robotic navigation of an interventional device (150) within a vascular region (130) during a current interventional device navigation procedure according to the method of claim 14;
controlling (S720) a robotic interventional device manipulator to navigate an interventional device (150) within a vascular region (130) during a current interventional device navigation procedure according to the method of claim 13; and
determining (S730) a difference between the simulated robotic navigation of the interventional device (150), and the navigation of the interventional device (150) performed by controlling the robotic interventional device manipulator to navigate the interventional device (150) within the vascular region (130) during the current interventional device navigation procedure.
